# EUROPEAN PATENT SPECIFICATION

(11) **EP 2 659 889 B1**
(45) Date of publication and mention of the grant of the patent: **26.04.2017**
(21) Application number: 11853993.1
(22) Date of filing: 28.12.2011
(51) Int. Cl.: A61K 31/46, A61K 31/192, A61K 9/16, A61K 9/20, A61K 9/28, A61K 47/32, A61K 47/36, A61K 47/38

(54) **ANTIPYRETIC/ANALGESIC COMPOSITION**
FIEBERSENKENDE/SCHMERZSTILLENDE ZUSAMMENSETZUNG
COMPOSITION ANTIPYRÉTIQUE/ANALGÉSIQUE

(30) Priority: 28.12.2010 JP 2010292812; 31.08.2011 JP 2011188208
(43) Date of publication of application: 06.11.2013
(73) Proprietor: Kowa Co., Ltd., Nagoya-shi, Aichi 460-8625 (JP)
(72) Inventor: KANO, Yuichiro, Fuji-shi Shizuoka 417-8650 (JP); SHIMOKAWA, Tatsuharu, Fuji-shi Shizuoka 417-8650 (JP)
(74) Representative: Blodig, Wolfgang
(86) International application number: PCT/JP2011/080383
(87) International publication number: WO 2012/091090

(56) References cited:
- EP-A1- 2 702 989
- WO-A1-2011/093498
- JP-A- 2000 026 313
- JP-A- 2000 344 665
- JP-A- 2005 289 906
- JP-A- 2006 225 367
- JP-A- 2007 091 633
- JP-A- 2007 284 423
- "KLUCEL®: HYDROXYPROPYLCELLULOSE, CHEMICAL AND PHYSICAL PROPERTIES", , 1986, XP002726117, Retrieved from the Internet: URL:http://legacy.library.ucsf.edu/documen tStore/c/n/f/cnf81a99/Scnf81a99.pdf [retrieved on 2014-06-23]
- ALVAREZ-LORENZO C ET AL: "Interactions between hydroxypropylcelluloses and vapour/liquid water", EUROPEAN JOURNAL OF PHARMACEUTICS AND BIOPHARMACEUTICS, ELSEVIER SCIENCE PUBLISHERS B.V., AMSTERDAM, NL, vol. 50, no. 2, September 2000 (2000-09), pages 307-318, XP004257206, ISSN: 0939-6411, DOI: 10.1016/S0939-6411(00)00104-1

## Description

### Technical Field

The present invention relates to a pharmaceutical composition comprising ibuprofen and butylscopolamine, which is useful for antipyresis and analgesia.

### Background Art

Ibuprofen is one kind of non-steroidal anti-inflammatory drugs (NSAIDs), and is known as an ethical drug which is effective for anti-inflammation, analgesia, and antipyresis, for example, in rheumatoid arthritis, arthralgia, arthritis, neuralgia, neuritis, backache and low back pain, cervicobrachial syndrome, uterine adnexitis, dysmenorrhea, and acute upper respiratory inflammation, and in post-operative and post-traumatic periods. Further, ibuprofen is also a compound which has undergone the so-called Rx-to-OTC switch, and is widely used as an OTC drug as well. In addition, an antipyretic and analgesic drug, and a combination cold remedy are commercially available in Japan as OTC drugs having ibuprofen blended with various drugs such as allylisopropylurea, isopropylantipyrine, ethenzamide, bromovalerylurea, and magnesium oxide (Non Patent Literatures 1 and 2).

Meanwhile, as one kind of the NSAIDs, ibuprofen may cause a gastrointestinal disorder and the like as side effects.

Therefore, there is a problem in that, even when an increase in amount of ibuprofen to be administered is sought in order to provide a sufficient antipyretic and analgesic effect, the administration amount cannot be increased.

As a solution to the problem, it is known that, when butylscopolamine bromide, which has an analgesic and spasmolytic action, is taken in combination with ibuprofen, an excellent analgesic action is exhibited even at a low dose (Patent Literature 1). Further, butylscopolamine bromide is also a compound which has undergone the Rx-to-OTC switch, and OTC drugs having efficacy and effects on gastralgia, abdominal pain, a cramp (colic or a spasm), hyperacidity, and heartburn are commercially available in Japan (Non Patent Literature 3).

As preparations having blended therein ibuprofen and butylscopolamine bromide, tablets and fine granules described in Patent Literature 1 are known.

However, it has not been known whether or not ibuprofen - butylscopolamine bromide interaction is shown.

### Citation List

### Patent Literature

[Patent Literature 1] JP-B-4153124

### Non Patent Literature

[Non-Patent Literature 1] OTC handbook, 2008-09, product information collection, Academic information distribution center corporation, pages 2 to 9
[Non-Patent Literature 2] OTC handbook, 2008-09, product information collection, Academic information distribution center corporation, pages 16 to 39
[Non-Patent Literature 3] OTC handbook, 2008-09, product information collection, Academic information distribution center corporation, pages 142 to 151

### Summary of Invention

### Technical Problem

The inventors of the present invention have made studies in order to formulate a pharmaceutical composition comprising ibuprofen and butylscopolamine bromide into a solid preparation. As a result, the inventors have found that, when ibuprofen and butylscopolamine bromide are stored as a mixture, an interaction unexpectedly occurs between these compounds, and that the interaction causes discoloration, wetting, and the like, with the result that it is difficult to keep a stable state during the storage.

Therefore, according to the present invention, provided is a stable pharmaceutical composition comprising ibuprofen and butylscopolamine bromide, and a stable solid preparation comprising the pharmaceutical composition.

### Solution to Problem

In view of the foregoing, the inventors of the present invention have made extensive studies on the storage stability of a pharmaceutical composition comprising ibuprofen and butylscopolamine bromide in order to solve the problem. As a result, the inventors have revealed that contact between ibuprofen and butylscopolamine bromide is a cause for the interaction, and have found that the interaction between ibuprofen and butylscopolamine bromide is suppressed by allowing a moisture-absorbing polymer to coexist in addition to ibuprofen and butylscopolamine bromide. The inventors have also found that the interaction is suppressed by containing ibuprofen and butylscopolamine bromide in a pharmaceutical composition in such a manner that these compounds are substantially free of contact with each other.

Accordingly, the present invention provides a pharmaceutical composition comprising ibuprofen, butylscopolamine bromide and a moisture-absorbing polymer, as defined in claim 1.

The pharmaceutical composition comprising ibuprofen, butylscopolamine bromide and a moisture-absorbing polymer of claim 1 may be a dosage form that comprises a solid preparation.

The present invention also provides a pharmaceutical composition comprising ibuprofen and butylscopolamine bromide, in such a manner that the ibuprofen and the butylscopolamine bromide are substantially free of contact with each other, as further defined in claim 3.

The pharmaceutical composition of claim 3 comprising ibuprofen and butylscopolamine bromide may be a dosage form that comprises a solid preparation.

Also described is an ameliorating agent for an interaction between ibuprofen and butylscopolamine bromide, the ameliorating agent comprising a moisture-absorbing polymer.

Also described is a method for ameliorating an interaction between ibuprofen and butylscopolamine bromide, the method comprises using a moisture-absorbing polymer.

The present invention also provides use of a moisture-absorbing polymer for ameliorating an interaction between ibuprofen and butylscopolamine bromide, as defined in claim 9.

Also described is a moisture-absorbing polymer for use in ameliorating an interaction between ibuprofen and butylscopolamine bromide.

### Effects of Invention

According to the present invention, the interaction between ibuprofen and butylscopolamine bromide can be ameliorated. Thus, the pharmaceutical composition comprising ibuprofen and butylscopolamine bromide, which is excellent in storage stability, can be provided.

Further, a tablet comprising ibuprofen and butylscopolamine bromide can be reduced in size. As a result, one tablet has only to be taken at a time for taking 150 mg of ibuprofen and 10 mg of butylscopolamine, while two tablets have had to be taken at a time heretofore. Accordingly, dose compliance can be significantly improved.

### Description of Embodiments

First, description is made of a pharmaceutical composition comprising ibuprofen, butylscopolamine bromide, and a moisture-absorbing polymer (hereinafter sometimes referred to as "composition A").

Ibuprofen to be used in the pharmaceutical composition is a known compound, and may be produced by a known method. Alternatively, a commercially available product thereof may be used. In the present invention, ibuprofen that is listed in an official compendium or the like established in a country, a region, or the like is preferred. Examples of the official compendium include the Japanese Pharmacopoeia, the US Pharmacopeia, the British Pharmacopoeia, the European Pharmacopoeia, and the Pharmacopoeia of the People's Republic of China. The description of the ibuprofen according to the Japanese Pharmacopoeia is white crystalline powder.

Further, the content of ibuprofen in the pharmaceutical composition has only to be determined with appropriate consideration of a daily dose depending on, for example, the sex, age, and condition of a recipient, and the daily dose in terms of ibuprofen is preferably such an amount that 10 to 3,000 mg can be taken, more preferably such an amount that 30 to 2,000 mg can be taken, even more preferably such an amount that 100 to 600 mg can be taken. The daily dose may be taken in one to four divided doses, and is preferably taken in three divided doses. A single dose in terms of ibuprofen is preferably 60 to 200 mg, more preferably 150 mg or 200 mg.

Butylscopolamine bromide to be used in the pharmaceutical composition is a known compound, and may be produced by a known method. Alternatively, a commercially available product thereof may be used. In the present invention, butylscopolamine bromide that is listed in an official compendium or the like established in a country, a region, or the like is preferred. Examples of the official compendium include the Japanese Pharmacopoeia, the US Pharmacopeia, the British Pharmacopoeia, the European Pharmacopoeia, and the Pharmacopoeia of the People's Republic of China. The description of the butylscopolamine bromide according to the Japanese Pharmacopoeia is a white crystal or crystalline powder.

Further, the content of butylscopolamine bromide in the pharmaceutical composition has only to be determined with appropriate consideration of a daily dose depending on, for example, the sex, age, and condition of the recipient, and the daily dose in terms of butylscopolamine bromide is preferably such an amount that 3 to 1,000 mg can be taken, more preferably such an amount that 5 to 500 mg can be taken, even more preferably such an amount that 30 to 100 mg can be taken per day. The daily dose may be taken in one to five divided doses, and is preferably taken in three divided doses. A single dose in terms of butylscopolamine bromide is preferably 10 to 20 mg, more preferably 10 mg or 20 mg at a time.

A content ratio between ibuprofen and butylscopolamine bromide contained in the pharmaceutical composition has only to be determined with appropriate consideration depending on the daily dose of each component described above, and the pharmaceutical composition contains butylscopolamine bromide at preferably 0.0125 to 1 part by mass, more preferably 0.022 to 1 part by mass with respect to 1 part by mass of ibuprofen.

Further, the content of ibuprofen in the pharmaceutical composition is preferably 35 to 95 mass%, more preferably 40 to 95 mass%, even more preferably 45 to 90 mass%, even more preferably 50 to 85 mass% with respect to the total mass of the pharmaceutical composition.

The lower limit of the content of butylscopolamine bromide in the pharmaceutical composition is preferably 0.1 mass% or more, more preferably 0.5 mass% or more, even more preferably 1 mass% or more, further more preferably 2 mass% or more, further more preferably 3 mass% or more, still more preferably 3.2 mass% or more, further more preferably 3.4 mass% or more with respect to the total mass of the pharmaceutical composition. On the other hand, the upper limit thereof is preferably 10 mass% or less, more preferably 8 mass% or less, even more preferably 6.5 mass%, further more preferably 6 mass%.

In the present invention, the moisture-absorbing polymer ameliorates an interaction between ibuprofen and butylscopolamine bromide.

The moisture-absorbing polymer to be contained in the pharmaceutical composition is a polymer having moisture-absorbing property and has an ameliorating effect on the interaction between ibuprofen and butylscopolamine bromide. Examples thereof include polymers such as a cellulose derivative having moisture-absorbing property or a salt thereof, a 1-vinyl-2-pyrrolidone polymerization product having moisture-absorbing property, and a starch derivative having moisture-absorbing property or a salt thereof. Of those, a cellulose derivative having moisture-absorbing property or a salt thereof is preferred.

Examples of the cellulose derivative having moisture-absorbing property or the salt thereof include a cellulose in which a hydroxy group is etherified, an ester of an etherified cellulose, and crosslinked polymerization products or salts thereof. Specific examples thereof include carmellose, carmellose potassium, carmellose calcium, carmellose sodium, croscarmellose sodium, hydroxyethylcellulose, and hydroxypropyl methyl cellulose acetate succinate. Of those, carmellose, carmellose potassium, carmellose calcium, carmellose sodium, and croscarmellose sodium are preferred.

In the embodiment of the invention of claim 1, the moisture-absorbing polymer is at least one selected from the group consisting of carmellose, carmellose potassium, carmellose calcium, carmellose sodium, and croscarmellose sodium.

Those compounds are known compounds, and may be produced by known methods. Alternatively, commercially available products thereof may be used.

Further, examples of the 1-vinyl-2-pyrrolidone polymerization product having moisture-absorbing property include a linear polymerization product of 1-vinyl-2-pyrrolidone and a crosslinked polymerization product of 1-vinyl-2-pyrrolidone. Specific examples of the 1-vinyl-2-pyrrolidone polymerization product include polyvinylpyrrolidone K17, polyvinylpyrrolidone K25, polyvinylpyrrolidone K30, polyvinylpyrrolidone K90, and crospovidone. Further, examples of the starch derivative having moisture-absorbing property or the salt thereof include carboxymethyl ether of starch or a salt thereof, and specific examples thereof include sodium carboxymethyl starch.

Further, the content of the moisture-absorbing polymer in the pharmaceutical composition has only to be determined with appropriate consideration based on, for example, the kind of the moisture-absorbing polymer to be used, in terms of ameliorating the interaction between ibuprofen and butylscopolamine bromide, and, for example, the pharmaceutical composition contains the moisture-absorbing polymer at preferably 0.01 to 3 parts by mass, more preferably 0.02 to 2 parts by mass with respect to 1 part by mass of ibuprofen.

The pharmaceutical composition may be formulated into various dosage forms in accordance with known methods described in, for example, the General Rules for Preparations of the Japanese Pharmacopoeia, Fifteenth Edition or Sixteenth Edition, with appropriate use of formulation additives.

The dosage form of the pharmaceutical composition, which is not particularly limited, is preferably a solid preparation from the viewpoints of, for example, ease of dosing and drug dose control. Specific examples of the solid preparation include: oral preparations such as capsules, pills, granules, fine granules, powders , tablets (including tablets for oral cavity application such as an oral dispersing tablet, a chewable tablet, a dispersible tablet, a solution tablet, a troche, a sublingual tablet, a buccal tablet, an adhesive tablet, and a gum tablet as well), a dry syrup, and an oral jelly; and parenteral preparations such as suppositories. Of those, an oral solid preparation is preferred. In addition, such pharmaceutical composition may be coated by a known method with, for example, sugar coating or film coating.

Next, description is made of a pharmaceutical composition comprising ibuprofen and butylscopolamine bromide in such a manner that these compounds are substantially free of contact with each other (hereinafter sometimes referred to as "composition B").

The phrase "comprising ... in such a manner that ... substantially free of contact with each other" means that the pharmaceutical composition comprises ibuprofen and butylscopolamine bromide in such a manner that these compounds are free of contact with each other so as not to express an interaction in the pharmaceutical composition. However, it is preferred to comprise ibuprofen and butylscopolamine bromide in such a manner that these compounds are free of contact with each other. Further, ibuprofen and butylscopolamine bromide to be contained in the composition B, their contents, and the like are the same as those in the composition A.

Further, the pharmaceutical composition (composition B) may be formulated into various dosage forms in accordance with known methods described in, for example, the General Rules for Preparations of the Japanese Pharmacopoeia, Fifteenth Edition or Sixteenth Edition, with appropriate use of formulation additives.

In addition, the dosage form of the pharmaceutical composition (composition B), which is not particularly limited, is preferably a solid preparation from the viewpoints of, for example, ease of dosing and drug dose control. Specific examples of the solid preparation include: oral preparations such as capsules, pills, granules, fine granules, powders, tablets (including tablets for oral cavity application such as an oral dispersing tablet, a chewable tablet, a dispersible tablet, a solution tablet, a troche, a sublingual tablet, a buccal tablet, an adhesive tablet, and a gum tablet as well), a dry syrup, and an oral jelly; and parenteral preparations such as suppositories. Of those, an oral solid preparation is preferred. In addition, such pharmaceutical composition may be coated by a known method with, for example, sugar coating or film coating.

As the solid preparation comprising ibuprofen and butylscopolamine bromide in such a manner that these compounds are substantially free of contact with each other, there is given one comprising: (A) ibuprofen itself or a solid composition comprising ibuprofen; and (B) butylscopolamine bromide itself or a solid composition comprising butylscopolamine bromide, in which components constituting these solid compositions allow ibuprofen and butylscopolamine bromide to be disposed in such a manner that these compounds are free of contact with each other. The form of each of the solid compositions is, for example, a powdery, particulate, or tablet-like form.

Specific forms of the above-mentioned solid preparation having ibuprofen and butylscopolamine bromide disposed in such a manner that these compounds are free of contact with each other may be exemplified by the following forms (I) to (VIII), each of which may be, as described above, produced and formulated by known methods, such as known methods described in the General Rules for Preparations of the Japanese Pharmacopoeia, Fifteenth Edition or Sixteenth Edition, with appropriate use of formulation additives.

(I) A solid preparation prepared by granulating any one of ibuprofen and butylscopolamine bromide by an appropriate method into a granular product, and incorporating the other of ibuprofen and butylscopolamine bromide, which has not been granulated, into the granular product (e.g., powders or granules, and a preparation obtained by further coating the granular product by an appropriate method).
(II) A solid preparation prepared by granulating ibuprofen and butylscopolamine bromide separately by appropriate methods into granular products and incorporating these granular products (e.g., powders or granules, and a preparation obtained by further coating the granular products by an appropriate method).
(III) Capsules obtained by filling powders, granules, or the like prepared in the above (I) or (II) into capsules.
(IV) Tablets obtained by tableting the granular product or the like prepared in the above (I) or (II) by an appropriate method. The tableting may be achieved by a compression method or, alternatively, by molding the granular product or the like into a given shape by an appropriate method.
(V) Multiple layer tablets prepared in such a manner that ibuprofen and butylscopolamine bromide are substantially free of contact with each other, and a preparation obtained by further coating the multiple layer tablets by an appropriate method. The multiple layer tablet is preferably one in which ibuprofen and butylscopolamine bromide are positioned in layers different from each other, more preferably a multilayered tablet including three or more layers in which a layer containing ibuprofen and a layer containing butylscopolamine bromide are positioned in such a manner that the layers are free of contact with each other. It should be noted that the granular product prepared in the above (I) or (II) may be used as each of the ibuprofen and the butylscopolamine bromide.
(VI) Dry-coated tablets prepared by disposing any one of ibuprofen and butylscopolamine bromide in its core tablet (also referred to as "center tablet" or "inner core"), in such a manner that ibuprofen and butylscopolamine bromide are substantially free of contact with each other, and a preparation obtained by further coating the dry-coated tablets by an appropriate method. It should be noted that the granular product prepared in the above (I) or (II) may be used as each of the ibuprofen and the butylscopolamine bromide.
(VII) A preparation using, in place of the granular product of the above (I) or (II), an inclusion compound(s) obtained by enclosing any one or both of ibuprofen and butylscopolamine bromide with, for example, any of cyclodextrins such as α-cyclodextrin, β-cyclodextrin, and γ-cyclodextrin.
(VIII) A preparation obtained by incorporating any one of ibuprofen and butylscopolamine bromide into a preparation prepared by a general method and providing thereto a sugar coating layer or a film coating layer, the sugar coating layer or the film coating layer containing the other compound, the preparation being prepared in such a manner that ibuprofen and butylscopolamine bromide are substantially free of contact with each other (referred to as "sugar-coated tablets" or "film-coated tablets" when the dosage form is a tablet).

The granular product in the above (I), (II), or the like may be prepared by a known granulation method such as extrusion granulation, oscillating granulation, stirring granulation, fluidized bed granulation, spray drying granulation, crushing granulation, or melt granulation, with appropriate use of formulation additives. In the present invention, the granular product containing ibuprofen and the granular product containing butylscopolamine bromide may both be prepared by the same granulation method, or may be prepared by different granulation methods.

For example, the granular product containing either ibuprofen or butylscopolamine bromide may be prepared by granulation with a mixture containing, for example, ibuprofen or butylscopolamine bromide, a fluidizing agent (e.g., light anhydrous silicic acid or hydrated silicon dioxide), and a binder (e.g., hydroxypropyl cellulose or hypromellose).

Alternatively, the granular product may be prepared by granulating ibuprofen or butylscopolamine bromide with a binder solution in which a fluidizing agent and the like are dispersed. Alternatively, the granular product may be produced by melting and granulating ibuprofen or butylscopolamine bromide through use of the following binder which is solid at normal temperature and has such a low melt point (solidifying point) as to melt or soften by heating, the melt point (solidifying point) being lower than the melt point (solidifying point) of ibuprofen or butylscopolamine bromide: MACROGOL (such as MACROGOL 4000, MACROGOL 6000, or MACROGOL 20000); oils and fats (e.g., hydrogenated tallow oil, hydrogenated oil, hydrogenated vegetable oil, hydrogenated soybean oil, carnauba wax, white beeswax, beeswax, and Japan wax); hydrocarbons (e.g., paraffin and microcrystalline wax); higher alcohols (e.g., cetyl alcohol and stearyl alcohol); fatty acids (e.g., stearic acid); and fatty acid esters (e.g., acetyl glycerin fatty acid ester, glycerin fatty acid ester, sucrose fatty acid ester, sorbitan fatty acid ester, and glyceryl monostearate).

It should be noted that the use amount of the fluidizing agent is generally 0.001 to 1 part by mass, preferably 0.002 to 0.5 part by mass with respect to 1 part by mass of the solid preparation. Further, the use amount of the binder is generally 0.001 to 3 parts by mass, preferably 0.005 to 2 parts by mass, more preferably 0.01 to 1 part by mass with respect to 1 part by mass of the solid preparation.

Further, a commercially available product may be used as the granular product containing ibuprofen, and examples thereof in Japan may include IBUPROFEN Granules 20% "TATSUMI" (Tatsumi Kagaku Co., Ltd.), BRUFEN (trademark) Gr. 20% (manufactured by KAKEN PHARMACEUTICAL CO., LTD.), and LANDELUN (trademark) Granules 20% (manufactured by Tsuruhara Pharmaceutical Co., Ltd.). It should be noted that, through the replacement of ibuprofen with butylscopolamine bromide, the granular product containing butylscopolamine bromide may be prepared in the same manner as above, or a commercially available product thereof may be used.

Further, a product obtained by further allowing a moisture-absorbing polymer to coexist in the pharmaceutical composition (composition B) may also be given as an aspect of the present invention. As described in Examples below, the moisture-absorbing polymer exhibits an effect of suppressing interaction between ibuprofen and butylscoporamine bromide, and hence the pharmaceutical composition allowed to contain ibuprofen and butylscopolamine bromide in such a manner that these compounds are substantially free of contact with each other preferably further contains the moisture-absorbing polymer. It should be noted that such moisture-absorbing polymer, its content, and the like are the same as those in the composition A.

In this case, specific forms of the pharmaceutical composition (composition B) containing the moisture-absorbing polymer in the case where the dosage form of the pharmaceutical composition is a solid preparation may be exemplified by the following forms (i) to (viii), which are based on the methods according to the above (I) to (VIII) and the like. As for a specific formulation method and the like, production and formulation may be performed, in the same manner as above, by known methods described in, for example, the General Rules for Preparations of the Japanese Pharmacopoeia, Fifteenth Edition or Sixteenth Edition, with appropriate use of formulation additives.
(i) A solid preparation prepared through use of ibuprofen, butylscopolamine bromide, and the moisture-absorbing polymer by granulating any one of ibuprofen and butylscopolamine bromide by an appropriate method into a granular product, and incorporating the other of ibuprofen and butylscopolamine bromide, which has not been granulated, into the granular product (e.g., powders or granules, and a preparation obtained by further coating the granular product by an appropriate method). The moisture-absorbing polymer may be incorporated into the granular product, or may be incorporated separately from the granular product.
(ii) A solid preparation prepared through use of ibuprofen, butylscopolamine bromide, and the moisture-absorbing polymer by granulating ibuprofen and butylscopolamine bromide separately by appropriate methods into granular products, and incorporating these granular products (e.g., powders or granules, and a preparation obtained by further coating the granular products by an appropriate method). The moisture-absorbing polymer may be incorporated into any one of the granular products, may be incorporated into both the granular products, or may be incorporated separately from the granular products.
(iii) Capsules obtained by filling powders, granules, or the like prepared in the above (i) or (ii) into capsules.
(iv) Tablets obtained by tableting the granular product or the like prepared in the above (i) or (ii) by an appropriate method. The tableting may be achieved by a compression method, or by molding the granular product or the like into a given shape by an appropriate method.
(v) Multilayered tablets prepared through use of ibuprofen, butylscopolamine bromide, and the moisture-absorbing polymer in such a manner that ibuprofen and butylscopolamine bromide are substantially free of contact with each other, and a preparation obtained by further coating the multiple layer tablet by an appropriate method. The multiple layer tablet is preferably one in which ibuprofen and butylscopolamine bromide are positioned in layers different from each other, more preferably a multiple layer tablet including three or more layers in which a layer containing ibuprofen and a layer containing butylscopolamine bromide are positioned in such a manner that the layers are free of contact with each other. It should be noted that the granular product prepared in the above (i) or (ii) may be used as each of ibuprofen and butylscopolamine bromide. In the multiple layer tablets, the moisture-absorbing polymer may be positioned in any one of the layer containing ibuprofen and the layer containing butylscopolamine bromide, or may be positioned in each of both the layers. In addition, the moisture-absorbing polymer may be positioned in an intermediate layer of any one of the layers.
(vi) Dry-coated tablet prepared through use of ibuprofen, butylscopolamine bromide, and the moisture-absorbing polymer by disposing any one of ibuprofen and butylscopolamine bromide in its core tablet (also referred to as "center tablet" or "inner core"), in such a manner that ibuprofen and butylscopolamine bromide are substantially free of contact with each other, and a preparation obtained by further coating the dry-coated tablets by an appropriate method. It should be noted that the granular product prepared in the above (i) or (ii) may be used as each of ibuprofen and butylscopolamine bromide. In the dry-coated tablets, the moisture-absorbing polymer may be positioned in the tablet core, may be positioned in an outer shell, or may be positioned in both the tablet core and the outer shell.
(vii) A preparation using ibuprofen, butylscopolamine bromide, and the moisture-absorbing polymer and using, in place of the granular product of the above (i) or (ii), an inclusion compound(s) obtained by enclosing any one or both of ibuprofen and butylscopolamine bromide with, for example, cyclodextrins such as α-cyclodextrin, β-cyclodextrin, and γ-cyclodextrin. The moisture-absorbing polymer may be positioned in the vicinity of any one of the inclusion compounds, or may be positioned in the vicinity of both the inclusion compounds.
(viii) A preparation obtained through use of ibuprofen, butylscopolamine bromide and the moisture-absorbing polymer and obtained by incorporating any one of ibuprofen and butylscopolamine bromide into a preparation prepared by a general method and providing thereto a sugar coating layer or a film coating layer, the sugar coating layer or the film coating layer containing the other of ibuprofen and butylscopolamine bromide, the preparation being prepared in such a manner that ibuprofen and butylscopolamine bromide are substantially free of contact with each other (referred to as "sugar-coated tablets" or "film-coated tablets" when the dosage form is a tablet). The moisture-absorbing polymer may be positioned in the preparation prepared by a general method, may be positioned in the sugar coating layer or the film coating layer, or may be positioned in both the sugar coating layer and the film coating layer. In addition, the moisture-absorbing polymer may be positioned in all of the preparation, the sugar coating layer, and the film coating layer.

In addition, the pharmaceutical compositions (composition A and composition B) of the present invention may each include one kind or two or more kinds selected from the group consisting of medicines except ibuprofen and butylscopolamine bromide, such as an antipyretic and analgesic drug, an antihistamine, an antitussive, noscapines, a bronchodilator, an expectorant, a hypnotic and sedative agent, vitamins, an anti-inflammatory agent, a gastric mucosa protective agent, an anticholinergic agent, crude drugs, a Chinese medicinal prescription, and a xanthine-based component.

Examples of the antipyretic and analgesic drug include aspirin, aspirin aluminum, acetaminophen, isopropylantipyrine, ethenzamide, sasapyrine, salicylamide, sodium salicylate, tiaramide hydrochloride, lactylphenetidine, and loxoprofen sodium hydrate.

Examples of the antihistamine include azelastine hydrochloride, alimemazine tartrate, isothipendyl hydrochloride, iproheptine hydrochloride, epinastine hydrochloride, emedastine fumarate, carbinoxamine diphenyldisulfonate, carbinoxamine maleate, clemastine fumarate, dl-chlorpheniramine maleate, d-chlorpheniramine maleate, ketotifen fumarate, difeterol hydrochloride, difeterol phosphate, diphenylpyraline hydrochloride, diphenylpyraline teoclate, diphenhydramine hydrochloride, diphenhydramine salicylate, diphenhydramine tannate, triprolidine hydrochloride, tripelennamine hydrochloride, thonzylamine hydrochloride, phenethazine hydrochloride, promethazine hydrochloride, promethazine methylene disalicylate, mequitazine, methdilazine hydrochloride, mebhydrolin napadisilate, and emedastine fumarate.

Examples of the antitussive include alloclamide hydrochloride, eprazinone hydrochloride, carbetapentane citrate, cloperastine hydrochloride, cloperastine fendizoate, codeine phosphate, dihydrocodeine phosphate, sodium dibunate, dimemorfan phosphate, dextromethorphan hydrobromide, dextromethorphan phenolphthalinate, tipepidine citrate, and tipepidine hibenzate.

Examples of the noscapines include noscapine hydrochloride and noscapine.

Examples of the bronchodilator include trimetoquinol hydrochloride, phenylpropanolamine hydrochloride, phenylephrine hydrochloride, pseudoephedrine hydrochloride, pseudoephedrine sulfate, methylephedrine, dl-methylephedrine hydrochloride, 1-methylephedrine hydrochloride, dl-methylephedrine saccharinate, and methoxyphenamine hydrochloride.

Examples of the expectorant include ambroxol hydrochloride, foeniculated ammonia spirit, ethylcysteine hydrochloride, ammonium chloride, carbocysteine, guaifenesin, potassium guaiacolsulfonate, potassium cresolsulfonate, bromhexine hydrochloride, methylcysteine hydrochloride, 1-menthol, and lysozyme hydrochloride.

Examples of the hypnotic and sedative agent include allylisopropylacetylurea and bromvalerylurea.

Examples of the vitamins include vitamin B₁, vitamin B₂, vitamin B₅, vitamin B₆, vitamin B₁₂, vitamin C, hesperidin and a derivative thereof, and salts thereof (e.g., thiamine, thiamine chloride hydrochloride, thiamine nitrate, dicethiamine hydrochloride, cetotiamine hydrochloride, fursultiamine, fursultiamine hydrochloride, octothiamine, cycothiamine, thiamine disulfide, bisibuthiamine, bisbentiamine, prosultiamine, benfotiamine, riboflavin, riboflavin phosphate, riboflavin butyrate, sodium riboflavin phosphate, panthenol, pantethine, calcium pantothenate, sodium pantothenate, pyridoxine hydrochloride, pyridoxal phosphate, cyanocobalamin, mecobalamin, ascorbic acid, sodium ascorbate, calcium ascorbate, and hesperidin).

Examples of the anti-inflammatory agent include glycyrrhizic acid and a derivative thereof, and salts thereof (e.g., dipotassium glycyrrhizinate and monoammonium glycyrrhizinate), seaprose, semialkaline proteinase, serrapeptase, tranexamic acid, proctase, pronase, and bromelain.

Examples of the gastric mucosa protective agent include aminoacetic acid, aldioxa, magnesium silicate, gefarnate, synthetic aluminum silicate, synthetic hydrotalcite, magnesium oxide, dihydroxyaluminum aminoacetate (aluminum glycinate), an aluminum hydroxide gel, an aluminium hydroxide-magnesium carbonate mixed dried gel, a coprecipitation product of aluminum hydroxide and sodium hydrogen carbonate, a coprecipitation product of aluminum hydroxide, calcium carbonate, and magnesium carbonate, a coprecipitation product of magnesium hydroxide and aluminum potassium sulfate, sucralfate, cetraxate hydrochloride, sofalcone, magnesium carbonate, teprenone, potassium copper chlorophyllin, sodium copper chlorophyllin, magnesium metasilicate aluminate, and methylmethionine sulfonium chloride.

Examples of the anticholinergic agent include oxyphencyclimine hydrochloride, dicyclomine hydrochloride, methixene hydrochloride, scopolamine hydrobromide, a Datura extract, timepidium bromide, methylatropine bromide, methylanisotropine bromide, methylscopolamine bromide, methyl-1-hyoscyamine bromide, methylbenactyzium bromide, pirenzepine hydrochloride, belladonna alkaloid, a belladonna extract, belladonna total alkaloid, isopropamide iodide, diphenylpiperidinomethyldioxolan iodide, a scopolia extract, scopolia rhizome, and scopolia Rhizome total alkaloid citrate.

Examples of the crude drugs include herbal medicines such as Mallotus bark, gambir, Epimedium herb, fennel, turmeric, Corydalis tuber, isodon japonicus, Scutellaria root, Polygonatum rhizome, Phellodendron bark, prunus jamasakura bark, Coptis rhizome, Polygala root, zedoary, Japanese valerian, chamomile, Trichosanthes seed, glycyrrhiza, Platycodon root, apricot kernel, Lycium fruit, Lycium leaf, Schizonepeta spike, cinnamon bark, Cassia seed, Gentian, Geranium herb, Cyperus rhizome, oriental bezoar, Schisandra fruit, Asiasarum root, Zanthoxylum fruit, Aster tataricus, Lycium bark, peony root, musk, Adenophora polymorpha, Plantago seed, Plantago herb, animal bile (including bear bile), ginger, herbal medicine prepared from dried earthworms, Magnolia flower, Lycoris radiata bulb, senega, Cnidium rhizome, Peucedanum root, Swertia herb, Atractylodes lancea rhizome, mulberry bark, Perilla herb, garlic, Panax japonicus rhizome, clove, citrus unshiu peel, Japanese angelica root, ipecac, Nandina domestica fruit, ginseng, fritillaria bulb, Ophiopogon tuber, Mentha herb, Pinellia tuber, saffron, Agkistrodon, Angelica dahurica root, Atractylodes rhizome, Poria sclerotium, Moutan bark, oyster shell, Ephedra herb and young deer horn and their extracts (including extracts, tincture and dried extracts).

Examples of the Chinese medicinal prescription include Kakkonto, Keishito, Kousosan, Saikokeishito, Shosaikoto, Shoseiryuto, Bakumondoto, Hangekobokuto and maoto.

Examples of the caffeines include caffeine and sodium benzoate, caffeine hydrate and anhydrous caffeine.

Examples of the xanthine derivatives include aminophylline, diprophylline, theophylline and proxyphylline.

In the present invention, as a preferred medicine, there are given, for example, medicines such as: antipyretic and analgesic drugs, e.g., acetaminophen, isopropylantipyrine, and ethenzamide; an antihistamine; hypnotic and sedative agents, e.g., allylisopropylacetylurea and bromvalerylurea; gastric mucosa protective agents, e.g., a dried aluminum hydroxide gel, synthetic hydrotalcite, and magnesium oxide; caffeines, e.g., caffeine and sodium benzoate, caffeine hydrate, and anhydrous caffeine, from the viewpoint that the pharmaceutical compositions (composition A and composition B) are used as an antipyretic and analgesic drug, a combination cold remedy, and the like.

In the present invention, which relates to a technology for ameliorating an interaction between ibuprofen and butylscopolamine bromide, the pharmaceutical compositions (composition A and composition B) are each not any of the following: tablets containing 75 mg of ibuprofen, 5 mg of butylscopolamine bromide, 10 mg of hydroxypropyl cellulose, 20 mg of croscarmellose sodium, 10 mg of light anhydrous silicic acid, 70 mg of crystalline cellulose, and 10 mg of talc per tablet (200 mg); and fine granules containing 150 mg of ibuprofen, 10 mg of butylscopolamine bromide, 610 mg of crystalline cellulose, 200 mg of carmellose calcium, 30 mg of hydroxypropyl cellulose, and 200 mg of Eudragit L, per packet (1.2 g). Preferably, the pharmaceutical compositions (composition A and composition B) are each not film-coated tablets having the tablet coated with a liquid obtained by dissolving 10 parts by mass of hydroxypropyl methyl cellulose (2910) and 1 part by mass of triethyl citrate in 115 parts by mass of purified water and dispersing 2 parts by mass of titanium oxide in the solution (having 5 mg of a film layer and weighing 205 mg per tablet).

In the case of the above-mentioned tablet weighing 200 mg per tablet, two tablets are required at a time for taking 150 mg of ibuprofen and 10 mg of butylscopolamine bromide. On the other hand, in the case of the pharmaceutical composition (tablet) according to the present invention, such doses can be achieved by taking one tablet at a time, and hence the pharmaceutical composition according to the present invention is excellent from the viewpoint of dose compliance.

It should be noted that the moisture-absorbing polymer in the interaction-ameliorating agent is the same as that in the composition A, and its concentration upon use is preferably 0.01 to 3 parts by mass, more preferably 0.02 to 2 parts by mass with respect to 1 part by mass of ibuprofen.

### Examples

Hereinafter, the present invention is described in more detail by way of examples. However, the present invention is by no means limited to these examples.

### Test Example 1: Study on interaction (1)

150 parts by mass of ibuprofen and 10 parts by mass of butylscopolamine bromide were mixed, and the mixture was placed into a glass vial and stored at 50°C (Reference Example 1). As controls for comparison, ibuprofen alone (Control Example 1) and butylscopolamine bromide alone (Control Example 2) were similarly placed into glass vials and stored at 50°C. States in the glass vials immediately after the start of the storage, 2 weeks thereafter, and 4 weeks thereafter were each evaluated. Table 1 shows the results.

**[Table 1]**

| | Immediately after start of storage | After 2 weeks | After 4 weeks |
|---|---|---|---|
| Control Example 1 | White powder | White powder | White powder |
| Control Example 2 | White powder | White powder | White powder |
| Reference Example 1 | White powder | White powder, wetted | Pale yellow powder, wetted |

As apparent from Table 1, it was found that, when the mixture of ibuprofen and butylscopolamine bromide was stored, the mixture wetted and discolored as well (Reference Example 1). On the other hand, in the case where each of ibuprofen and butylscopolamine bromide was stored alone, no change occurred (Control Examples 1 and 2). The results revealed that the change in the state of the mixture resulted from the interaction caused by ibuprofen and butylscopolamine bromide.

### Test Example 2: Study on interaction (2)

150 parts by mass of ibuprofen and 10 parts by mass of butylscopolamine bromide were mixed, and the mixture was placed into a glass vial and stored at 40°C (Reference Example 2). As controls for comparison, ibuprofen alone (Control Example 3) and butylscopolamine bromide alone (Control Example 4) were similarly placed into glass vials and stored at 40°C.

150 parts by mass of ibuprofen, 10 parts by mass of butylscopolamine bromide, and 20 parts by mass of croscarmellose sodium as an example of the moisture-absorbing polymer of the present invention were mixed, and the mixture was similarly placed into a glass vial and stored at 40°C (Example 1).

Further, 20 parts by mass of low substituted hydroxypropylcellulose as to exhibit no moisture-absorbing property were mixed in place of 20 parts by mass of croscarmellose sodium, and the mixture was similarly placed into a glass vial and stored at 40°C (Comparative Example 1).

States in the glass vials immediately after the start of the storage and 6 months thereafter were each evaluated. Table 2 shows the results.

**[Table 2]**

| | Immediately after start of storage | After 6 months |
|---|---|---|
| Control Example 3 | White powder | White powder |
| Control Example 4 | White powder | White powder |
| Reference Example 2 | White powder | Pale yellow powder, wetted |
| Example 1 | White powder | White powder |
| Comparative Example 1 | White powder | White wetted powder |

As apparent from Table 2, when the mixture of ibuprofen and butylscopolamine bromide was stored for 6 months, the mixture was in a wetted and discolored state (Reference Example 2). On the other hand, in the case where each of ibuprofen and butylscopolamine bromide was stored alone, the state immediately after the start of the storage was kept and no change occurred (Control Examples 3 and 4). The results revealed that the change in the state of the mixture resulted from the interaction caused by ibuprofen and butylscopolamine bromide.

Further, in the case where croscarmellose sodium was further added to the mixture of ibuprofen and butylscopolamine bromide, the state immediately after the start of the storage was kept and no change occurred (Example 1). On the other hand, in the case where low substituted hydroxypropylcellulose was further added to the mixture of ibuprofen and butylscopolamine bromide, the mixture wetted after the 6-month storage (Comparative Example 1).

Although croscarmellose sodium and low substituted hydroxypropylcellulose are both cellulose derivative-based polymers, the former exhibits moisture-absorbing property while the latter exhibits no moisture-absorbing property. Accordingly, the comparison between the results of Example 1 and Comparative Example 1 revealed that the interaction between ibuprofen and butylscopolamine bromide was able to be ameliorated to a greater extent when the polymer was a moisture-absorbing one.

### Test Example 3: Study on interaction (3)

150 parts by mass of ibuprofen and 10 parts by mass of butylscopolamine bromide were mixed, and the mixture was placed into a glass vial and stored at 60°C (Reference Example 3).

150 parts by mass of ibuprofen, 15 parts bymass of hydroxypropyl methyl cellulose, and 6 parts by mass of light anhydrous silicic acid were mixed, and the mixture was kneaded and granulated with purified water to yield a granulated product. The resultant granulatedproduct was mixed with 10 parts bymass of butylscopolamine bromide, and the mixture was placed into a glass vial and stored at 60°C (Example 2).

10 parts by mass of butylscopolamine bromide, 1 part by mass of hydroxypropyl cellulose, and 25.5 parts bymass of lactose hydrate were mixed, and the mixture was kneaded and granulated with purified water to yield a granulated product. The resultant granulated product was mixed with 150 parts bymass of ibuprofen, and the mixture was placed into a glass vial and stored at 60°C (Example 3).

150 parts bymass of ibuprofen, 15 parts by mass of hydroxypropyl methyl cellulose, and 6 parts by mass of light anhydrous silicic acid were mixed, and the mixture was kneaded and granulated with purified water to yield a granulated product. Meanwhile, 10 parts by mass of butylscopolamine bromide, 1 part by mass of hydroxypropyl cellulose, and 25.5 parts by mass of lactose hydrate were mixed, and the mixture was kneaded and granulated with purified water to yield a granulated product. The resultant two kinds of granulated products were mixed, and the mixture was placed into a glass vial and stored at 60°C (Example 4).

States in the glass vials immediately after the start of the storage and 1 week thereafter were each evaluated. Table 3 shows the results.

**[Table 3]**

| | Immediately after start of storage | After 1 week |
|---|---|---|
| Reference Example 3 | White powder | White powder solidified |
| Example 2 | White powder | White powder |
| Example 3 | White powder | White powder |
| Example 4 | White powder | White powder |

As apparent from Table 3, when the mixture of ibuprofen and butylscopolamine bromide was stored, the mixture fell into a solidified state in 1 week (Reference Example 3).

On the other hand, in each of the cases of containing ibuprofen and butylscopolamine bromide in such a manner that they were substantially free of contact with each other, the state immediately after the start of the storage was kept and no change occurred (Examples 2 to 4).

Thus, the results of Reference Example 3 and Examples 2 to 4 revealed that the interaction between ibuprofen and butylscopolamine bromide was able to be ameliorated by allowing ibuprofen and butylscopolamine bromide to be substantially free of contact with each other.

### Production Example 1

150 parts bymass of ibuprofen, 12 parts bymass of hydroxypropyl methyl cellulose, 25 parts by mass of croscarmellose sodium, and 6 parts by mass of light anhydrous silicic acid were mixed, and the mixture was kneaded and granulated with purified water, followed by grain sizing, to yield a granulated product. Meanwhile, 10 parts by mass of butylscopolamine bromide, 1 part by mass of hydroxypropyl cellulose, and 36 parts by mass of lactose hydrate were mixed, and the mixture was kneaded and granulated with ethanol, followed by grain sizing, to yield a granulated product. The resultant two kinds of granulated products were mixed with the addition of 10 parts by mass of talc to yield granules for compression tableting.

The resultant granules for compression tableting were subjected to compression tableting to yield tablets containing 150 mg of ibuprofen and 10 mg of butylscopolamine bromide per tablet.

### Production Example 2

10 parts by mass of hydroxypropyl methyl cellulose and 1 part by mass of triethyl citrate were dissolved in 115 parts by mass of purified water, and 2 parts by mass of titanium oxide were dispersed in the solution to prepare a film coating liquid. The coating liquid was sprayed with a coating apparatus to yield film-coated tablets having 10 mg of a film layer on the tablet (uncoated tablet) obtained in Production Example 1.

### Production Example 3

Tablets containing 150 mg of ibuprofen and 10 mg of butylscopolamine bromide per tablet was obtained in the same manner as in Production Example 1 except for using carmellose in place of croscarmellose sodium.

### Production Example 4

Film-coated tablets having 10 mg of a film layer on the tablet (uncoated tablet) obtained in Production Example 3 was obtained in the same manner as in Production Example 2.

### Test Example 4: Study on interaction (4)

The tablets (uncoated tablets) obtained in Production Example 1 and Production Example 3, and the film-coated tablets obtained in Production Example 2 and Production Example 4 were each placed into a glass vial and stored at 40°C.

The states of each tablet in the glass vial immediately after the start of the storage and 6 months thereafter were evaluated. Table 4 shows the results.

**[Table 4]**

| Composition of tablet (per tablet; only partly shown) | Production Example 1 | Production Example 2 | Production Example 3 | Production Example 4 |
|---|---|---|---|---|
| Ibuprofen | 150 mg | 150 mg | 150 mg | 150 mg |
| Butylscopolamine bromide | 10 mg | 10 mg | 10 mg | 10 mg |
| Croscarmellose sodium | 25 mg | 25 mg | - | - |
| Carmellose | - | - | 25 mg | 25 mg |
| Film layer | - | 10 mg | - | 10 mg |
| Immediately after start of storage | White tablet | White tablet | White tablet | White tablet |
| After 6 months at 40°C | White tablet | White tablet | White tablet | White tablet |

As apparent from Table 4, in the case of the tablet (uncoated tablet) containing ibuprofen, butylscopolamine bromide, and croscarmellose sodium as a cellulose derivative exhibiting moisture-absorbing property, the state immediately after the start of the storage was kept even after the 6-month storage and no change occurred (Production Example 1). Similarly, also in the case of the tablet (uncoated tablet) containing carmellose as a cellulose derivative exhibiting moisture-absorbing property in place of croscarmellose sodium, the state immediately after the start of the storage was kept even after the 6-month storage and no change occurred (Production Example 3).

Further, in the case of each of the tablets obtained by providing the respective uncoated tablets of Production Example 1 and Production Example 3 with film coatings, the state immediately after the start of the storage was kept even after the 6-month storage and no change occurred (Production Example 2 and Production Example 4).

### Production Example 5

200 parts bymass of ibuprofen, 12 parts bymass of hydroxypropyl methyl cellulose, 25 parts by mass of carmellose, and 6 parts by mass of light anhydrous silicic acid were mixed, and the mixture was kneaded and granulated with purified water, followed by grain sizing, to yield a granulated product. Meanwhile, 10 parts by mass of butylscopolamine bromide, 1 part by mass of hydroxypropyl cellulose, and 36 parts by mass of lactose hydrate were mixed, and the mixture was kneaded and granulated with ethanol, followed by grain sizing, to yield a granulated product. The resultant two kinds of granulated products were mixed with the addition of 10 parts by mass of talc to yield granules for compression tableting.

The resultant granules for compression tableting were subjected to compression tableting to yield tablets containing 200 mg of ibuprofen and 10 mg of butylscopolamine bromide per tablet.

### Production Example 6

10 parts by mass of hydroxypropyl methyl cellulose and 1 part by mass of triethyl citrate were dissolved in 115 parts by mass of purified water, and 2 parts bymass of titanium oxide were dispersed in the solution to prepare a film coating liquid. The coating liquid was sprayed with a coating apparatus to yield film-coated tablets having 10 mg of a film layer on the tablet (uncoated tablet) obtained in Production Example 5.

### Production Example 7

Tablets containing 200 mg of ibuprofen and 10 mg of butylscopolamine bromide per tablet was obtained in the same manner as in Production Example 5 except for using croscarmellose sodium in place of 25 parts by mass of carmellose.

### Production Example 8

Film-coated tablets having 10 mg of a film layer on the tablet (uncoated tablet) obtained in Production Example 7 was obtained in the same manner as in Production Example 6.

### Production Example 9

200 parts by mass of ibuprofen, 12 parts bymass of hydroxypropyl methyl cellulose, 25 parts by mass of carmellose, and 6 parts by mass of light anhydrous silicic acid were mixed, and the mixture was kneaded and granulated with purified water, followed by grain sizing, to yield a granulated product. Meanwhile, 20 parts by mass of butylscopolamine bromide, 1 part by mass of hydroxypropyl cellulose, and 26 parts by mass of lactose hydrate were mixed, and the mixture was kneaded and granulated with ethanol, followed by grain sizing, to yield a granulated product. The resultant two kinds of granulated products were mixed with the addition of 10 parts by mass of talc to yield granules for compression tableting.

The resultant granules for compression tableting were subjected to compression tableting to yield tablets containing 200 mg of ibuprofen and 20 mg of butylscopolamine bromide per tablet.

### Production Example 10

10 parts by mass of hydroxypropyl methyl cellulose and 1 part by mass of triethyl citrate were dissolved in 115 parts by mass of purified water, and 2 parts by mass of titanium oxide were dispersed in the solution to prepare a film coating liquid. The coating liquid was sprayed with a coating apparatus to yield film-coated tablets having 10 mg of a film layer on the tablet (uncoated tablet) obtained in Production Example 9.

### Production Example 11

Tablets containing 200 mg of ibuprofen and 20 mg of butylscopolamine bromide per tablet was obtained in the same manner as in Production Example 9 except for using croscarmellose sodium in place of 25 parts by mass of carmellose.

### Production Example 12

Film-coated tablets having 10 mg of a film layer on the tablet (uncoated tablet) obtained in Production Example 11 was obtained in the same manner as in Production Example 10.

### Industrial Applicability

According to the present invention, the storage-stable pharmaceutical composition comprising ibuprofen and butylscopolamine bromide can be provided.

Further, the composition can be provided as a pharmaceutical composition capable of containing a usual single dose (150 mg or 200 mg of ibuprofen and 10 mg or 20 mg of butylscopolamine bromide) (e.g., tablets containing the usual doses of the respective components per tablet), and hence is favorable for compliance by the recipient. Further, ibuprofen and butylscopolamine bromide can be easily used in combination, and hence the composition has an excellent analgesic action and is excellent as an antipyretic analgesic or combination cold remedy having reduced side effects.

## Claims

1. A pharmaceutical composition, comprising:
ibuprofen;
butylscopolamine bromide; and
a moisture-absorbing polymer,
wherein the moisture-absorbing polymer is at least one selected from the group consisting of carmellose, carmellose potassium, carmellose calcium, carmellose sodium and croscarmellose sodium,
provided that the pharmaceutical composition does not comprise tablets containing 75 mg of ibuprofen, 5 mg of butylscopolamine bromide, 10 mg of hydroxypropylcellulose, 20 mg of croscarmellose sodium, 10 mg of light anhydrous silicic acid, 70 mg of crystalline cellulose, and 10 mg of talc per tablet of 200 mg, and fine granules containing 150 mg of ibuprofen, 10 mg of butylscopolamine bromide, 610 mg of crystalline cellulose, 200 mg of carmellose calcium, 30 mg of hydroxypropylcellulose, and 200 mg of Eudragit L per packet of 1.2 g.

2. The pharmaceutical composition according to claim 1, wherein a dosage form of the pharmaceutical composition comprises a solid preparation.

3. A pharmaceutical composition, comprising:
ibuprofen; and
butylscopolamine bromide,
in such a manner that ibuprofen and butylscopolamine bromide are substantially free of contact with each other,
provided that the pharmaceutical composition does not comprise tablets containing 75 mg of ibuprofen, 5 mg of butylscopolamine bromide, 10 mg of hydroxypropylcellulose, 20 mg of croscarmellose sodium, 10 mg of light anhydrous silicic acid, 70 mg of crystalline cellulose, and 10 mg of talc per tablet of 200 mg, and fine granules containing 150 mg of ibuprofen, 10 mg of butylscopolamine bromide, 610 mg of crystalline cellulose, 200 mg of carmellose calcium, 30 mg of hydroxypropylcellulose, and 200 mg of Eudragit L per packet of 1.2 g.

4. The pharmaceutical composition according to claim 3, wherein the pharmaceutical composition comprises:
a granular product comprising ibuprofen; and
butylscopolamine bromide,
in such a manner that ibuprofen and butylscopolamine bromide are substantially free of contact with each other.

5. The pharmaceutical composition according to claim 3, wherein the pharmaceutical composition comprises:
a granular product comprising butylscopolamine bromide; and
ibuprofen,
in such a manner that ibuprofen and butylscopolamine bromide are substantially free of contact with each other.

6. The pharmaceutical composition according to claim 3, wherein the pharmaceutical composition comprises:
a granular product comprising ibuprofen; and
a granular product comprising butylscopolamine bromide,
in such a manner that ibuprofen and butylscopolamine bromide are substantially free of contact with each other.

7. The pharmaceutical composition according to any one of claims 3 to 6, further comprising a moisture-absorbing polymer,
wherein the moisture-absorbing polymer is at least one selected from the group consisting of carmellose, carmellose potassium, carmellose calcium, carmellose sodium and croscarmellose sodium.

8. The pharmaceutical composition according to any one of claims 3 to 7, wherein a dosage form of the pharmaceutical composition comprises a solid preparation.

9. Use of a moisture-absorbing polymer for ameliorating an interaction between ibuprofen and butylscopolamine bromide, wherein the moisture-absorbing polymer is at least one selected from the group consisting of carmellose, carmellose potassium, carmellose calcium, carmellose sodium and croscarmellose sodium.

## Patentansprüche

1. Pharmazeutische Zusammensetzung, umfassend:
Ibuprofen;
Butylscopolaminbromid; und
ein Feuchtigkeit absorbierendes Polymer,
wobei das Feuchtigkeit absorbierende Polymer wenigstens eines ist, ausgewählt aus der Gruppe, bestehend aus Carmellose, Carmellose-Kalium, Carmellose-Calcium, Carmellose-Natrium und Croscarmellose-Natrium,
mit der Maßgabe, dass die pharmazeutische Zusammensetzung keine Tabletten, die 75 mg Ibuprofen, 5 mg Butylscopolaminbromid, 10 mg Hydroxypropylcellulose, 20 mg Croscarmellose-Natrium, 10 mg leichte wasserfreie Kieselsäure, 70 mg kristalline Cellulose und 10 mg Talk pro Tablette von 200 mg enthalten, und feines Granulat, das 150 mg Ibuprofen, 10 mg Butylscopolaminbromid, 610 mg kristalline Cellulose, 200 mg Carmellose-Calcium, 30 mg Hydroxypropylcellulose und 200 mg Eudragit L pro Packung von 1,2 g enthält, umfasst.

2. Pharmazeutische Zusammensetzung nach Anspruch 1, wobei die Dosierungsform der pharmazeutischen Zusammensetzung eine feste Darreichungsform umfasst.

3. Pharmazeutische Zusammensetzung, umfassend:
Ibuprofen; und
Butylscopolaminbromid,
auf eine solche Weise, dass das Ibuprofen und das Butylscopolaminbromid im Wesentlichen keinen Kontakt zueinander haben,
mit der Maßgabe, dass die pharmazeutische Zusammensetzung keine Tabletten, die 75 mg Ibuprofen, 5 mg Butylscopolaminbromid, 10 mg Hydroxypropylcellulose, 20 mg Croscarmellose-Natrium, 10 mg leichte wasserfreie Kieselsäure, 70 mg kristalline Cellulose und 10 mg Talk pro Tablette von 200 mg enthalten, und feines Granulat, das 150 mg Ibuprofen, 10 mg Butylscopolaminbromid, 610 mg kristalline Cellulose, 200 mg Carmellose-Calcium, 30 mg Hydroxypropylcellulose und 200 mg Eudragit L pro Packung von 1,2 g enthält, umfasst.

4. Pharmazeutische Zusammensetzung nach Anspruch 3, wobei die pharmazeutische Zusammensetzung folgendes umfasst:
ein granuläres Produkt, das Ibuprofen umfasst; und
Butylscopolaminbromid,
auf eine solche Weise, dass das Ibuprofen und das Butylscopolaminbromid im Wesentlichen keinen Kontakt zueinander haben.

5. Pharmazeutische Zusammensetzung nach Anspruch 3, wobei die pharmazeutische Zusammensetzung folgendes umfasst:
ein granuläres Produkt, das Butylscopolaminbromid umfasst; und
Ibuprofen,
auf eine solche Weise, dass das Ibuprofen und das Butylscopolaminbromid im Wesentlichen keinen Kontakt zueinander haben.

6. Pharmazeutische Zusammensetzung nach Anspruch 3, wobei die pharmazeutische Zusammensetzung folgendes umfasst:
ein granuläres Produkt, das Ibuprofen umfasst; und
ein granuläres Produkt, das Butylscopolaminbromid umfasst;
auf eine solche Weise, dass das Ibuprofen und das Butylscopolaminbromid im Wesentlichen keinen Kontakt zueinander haben.

7. Pharmazeutische Zusammensetzung nach einem der Ansprüche 3 bis 6, ferner ein Feuchtigkeit absorbierendes Polymer umfassend, wobei das Feuchtigkeit absorbierende Polymer wenigstens eines ist, ausgewählt aus der Gruppe, bestehend aus Carmellose, Carmellose-Kalium, Carmellose-Calcium, Carmellose-Natrium und Croscarmellose-Natrium.

8. Pharmazeutische Zusammensetzung nach einem der Ansprüche 3 bis 7, wobei die Dosierungsform der pharmazeutischen Zusammensetzung eine feste Darreichungsform umfasst.

9. Verwendung eines Feuchtigkeit absorbierenden Polymers zur Verbesserung der Wechselwirkung zwischen Ibuprofen und Butylscopolaminbromid, wobei das Feuchtigkeit absorbierende Polymer wenigstens eines ist, ausgewählt aus der Gruppe, bestehend aus Carmellose, Carmellose-kalium, Carmellose-calcium, Carmellose-natrium und Croscarmellose-natrium.

## Revendications

1. Composition pharmaceutique comprenant :
de l'ibuprofène ;
du bromure de butylscopolamine ; et
un polymère absorbant l'humidité,
dans laquelle le polymère absorbant l'humidité est au moins l'un choisi dans l'ensemble constitué par la carmellose, la carmellose potassique, la carmellose calcique, la carmellose sodique et la croscarmellose sodique,
sous réserve que la composition pharmaceutique ne comprenne pas de comprimés contenant 75 mg d'ibuprofène, 5 mg de bromure de butylscopolamine, 10 mg d'hydroxypropylcellulose, 20 mg de croscarmellose sodique, 10 mg d'acide silicique anhydre léger, 70 mg de cellulose cristalline, et 10 mg de talc par comprimé de 200 mg, et de granules fins contenant 150 mg d'ibuprofène, 10 mg de bromure de butylscopolamine, 610 mg de cellulose cristalline, 200 mg de carmellose sodique, 30 mg d'hydroxypropylcellulose, et 200 mg d'Eudragit L par paquet de 1,2 g.

2. Composition pharmaceutique selon la revendication 1, dans laquelle une forme posologique de la composition pharmaceutique comprend une préparation solide.

3. Composition pharmaceutique comprenant :
de l'ibuprofène ; et
du bromure de butylscopolamine,
de telle manière que l'ibuprofène et le bromure de butylscopolamine soient pratiquement exempt de tout contact l'un avec l'autre,
sous réserve que la composition pharmaceutique ne comprenne pas de comprimés contenant 75 mg d'ibuprofène, 5 mg de bromure de butylscopolamine, 10 mg d'hydroxypropylcellulose, 20 mg de croscarmellose sodique, 10 mg d'acide silicique anhydre léger, 70 mg de cellulose cristalline, et 10 mg de talc par comprimé de 200 mg, et de granules fins contenant 150 mg d'ibuprofène, 10 mg de bromure de butylscopolamine, 610 mg de cellulose cristalline, 200 mg de carmellose calcique, 30 mg d'hydroxypropylcellulose, et 200 mg d'Eudragit L par paquet de 1,2 g.

4. Composition pharmaceutique selon la revendication 3, laquelle composition pharmaceutique comprend :
un produit granulaire comprenant de l'ibuprofène ; et
du bromure de butylscopolamine,
de telle manière que l'ibuprofène et le bromure de butylscopolamine soient pratiquement exempt de tout contact l'un avec l'autre.

5. Composition pharmaceutique selon la revendication 3, laquelle composition pharmaceutique comprend :
un produit granulaire comprenant du bromure de butylscopolamine ; et
de l'ibuprofène,
de telle manière que l'ibuprofène et le bromure de butylscopolamine soient pratiquement exempt de tout contact l'un avec l'autre.

6. Composition pharmaceutique selon la revendication 3, laquelle composition pharmaceutique comprend :
un produit granulaire comprenant de l'ibuprofène ; et
un produit granulaire comprenant du bromure de butylscopolamine,
de telle manière que l'ibuprofène et le bromure de butylscopolamine soient pratiquement exempt de tout contact l'un avec l'autre.

7. Composition pharmaceutique selon l'une quelconque des revendications 3 à 6, comprenant en outre un polymère absorbant l'humidité, dans laquelle le polymère absorbant l'humidité est au moins l'un choisi dans l'ensemble constitué par la carmellose, la carmellose potassique, la carmellose calcique, la carmellose sodique et la croscarmellose sodique.

8. Composition pharmaceutique selon l'une quelconque des revendications 3 à 7, dans laquelle une forme posologique de la composition pharmaceutique comprend une préparation solide.

9. Utilisation d'un polymère absorbant l'humidité pour améliorer une interaction entre l'ibuprofène et le bromure de butylscopolamine, dans laquelle le polymère absorbant l'humidité est au moins l'un choisi dans l'ensemble constitué par la carmellose, la carmellose potassique, la carmellose calcique, la carmellose sodique et la croscarmellose sodique.
